# EUROPÄISCHE PATENTSCHRIFT

(11) **EP 2 890 725 B1**
(45) Veröffentlichungstag und Bekanntmachung des Hinweises auf die Patenterteilung: **25.09.2019**
(21) Anmeldenummer: 13756047.0
(22) Anmeldetag: 22.08.2013
(51) Int. Cl.: C08G 18/28, C08G 18/32, C08G 18/38, C07D 317/36, C07D 319/06

(54) **ISOCYANATFUNKTIONELLE CYCLISCHE CARBONATE**
ISOCYANATE FUNCTIONAL CYCLIC CARBONATES
CARBONATE CYCLIQUE À ISOCYANATE FONCTIONNEL

(30) Priorität: 28.08.2012 EP 12182067
(43) Veröffentlichungstag der Anmeldung: 08.07.2015
(73) Patentinhaber: Covestro Deutschland AG, 51373 Leverkusen (DE)
(72) Erfinder: LAAS, Hans-Josef, 51519 Odenthal (DE)
(74) Vertreter: Levpat
(86) Internationale Anmeldenummer: PCT/EP2013/067464
(87) Internationale Veröffentlichungsnummer: WO 2014/033046

(56) Entgegenhaltungen:
- EP-A2- 0 328 150
- US-A- 3 072 613

## Beschreibung

Die Erfindung betrifft ein Verfahren zur Herstellung Isocyanatgruppen und cyclische Carbonatstrukturen enthaltender Verbindungen, die nach diesem Verfahren erhältlichen Produkte sowie eine Zusammensetzung enthaltend Isocyanatgruppen und cyclische Carbonatstrukturen enthaltenden Verbindungen. Des Weiteren betrifft die Erfindung die Verwendung der nach dem erfindungsgemäßen Verfahren erhältlichen Produkte und der erfindungsgemäßen Zusammensetzung als Ausgangskomponente bei der Herstellung von cyclische Carbonatstrukturen enthaltenden Polyurethanen, vernetzbaren Bindemitteln, sowie Lack-, Dichtstoff- oder Klebstoffrohstoffen.

Die Umsetzung cyclischer Carbonate mit Aminen zu Urethangruppen ist lange bekannt (z. B. US 3 072 613, US 3 084 140). Kombiniert man Verbindungen, die mindestens zwei cyclische Carbonatgruppen aufweisen, mit Polyaminen kann diese Reaktion zum Aufbau von Polyurethanen genutzt werden.

Eine sehr einfache, häufig genutzte Methode zur Herstellung von Verbindungen mit mehreren cyclischen Carbonatgruppen im Molekül besteht in der Umsetzung niedermolekularer hydroxyfunktioneller cyclischer Carbonate mit Polyisocyanaten oder isocyanatfunktionellen Prepolymeren.

Beispielsweise beschreibt die WO 2006/010408 isocyanatgruppenfreie Umsetzungsprodukte linearer Polyurethanpräpolymere auf Basis von Diphenylmethandiisocyanat (MDI) mit 4-(Hydroxymethyl)-1,3-dioxolan-2-on (Glycerincarbonat), die sich mit Verbindungen, die mindestens zwei primäre oder sekundäre Aminogruppen tragen, bereits bei Raumtemperatur vernetzen lassen. Solche Zweikomponenten-Bindemittel finden als Kleb- und Dichtstoffe, insbesondere als Kaschierklebstoff für Verbundfolien, Verwendung.

Mit hydroxyfunktionellen cyclischen Carbonaten blockierte Polyisocyanate, beispielsweise Umsetzungsprodukte von Polyisocyanuratpolyisocyanaten des 1,6-Diisocyanatohexan (Hexamethylendiisocyanat, HDI) mit Glycerincarbonat, sind Gegenstand der WO 2008/125419. In speziellen Acetalen, wie insbesondere 1,1,2,2-Dimethoxyethan, gelöst, härten solche Glycerincarbonat/Polyisocyanat-Addukte mit Polyetherpolyaminen oder Polyamidaminen ebenfalls bereits bei Raumtemperatur zu Lackfilmen hoher optischer Qualität aus.

Verbindungen, die cyclische Carbonatgruppen tragen, lassen sich auch mit hydroxyfunktionellen Reaktionspartnern kombinieren. Während die aminische Härtung der cyclischen Carbonatgruppen bereits bei niedrigen Temperaturen ausreichend schnell verläuft, erfolgt eine Vernetzung in diesem Fall allerdings nur bei erhöhten Temperaturen, in der Regel unter Einbrennbedingungen, und in Gegenwart spezieller Katalysatoren.

EP-A 0 911 352 beschreibt beispielsweise bei Raumtemperatur lagerstabile Einkomponentensysteme, bestehend aus Polyolen, Umsetzungsprodukten von Polyisocyanuratpolyisocyanaten des HDI und/oder des 1-Isocyanato-3,3,5-trimethyl-5-isocyanatomethylcyclohexans (Isophorondiisocyanat, IPDI) mit 5-(Hydroxymethyl)-5-ethyl-1,3-dioxan-2-on (TMP-Carbonat) als Vernetzerkomponenten und speziellen Metallcarboxylaten als Aushärtekatalysatoren, die ohne Abspaltung flüchtiger Verbindungen bei erhöhten Temperaturen zu harten lösemittelbeständigen Beschichtungen eingebrannt werden können.

Die Herstellung 1,3-Dioxan-2-on-Gruppen enthaltender Oligourethane aus Diisocyanaten oder Polyisocyanaten unter Verwendung von TMP-Carbonat und ihre Verwendung als Vernetzer für Polyole in thermisch härtbaren Beschichtungssystemen ist auch aus EP-A 0 703 230 bekannt. Das Dokument EP 0 328 150 A2 offenbart im Beispiel 18 die Herstellung einer Verbindung aus 1,5 Mol-Äquivalent HDI und 0,24 Mol-Äquivalent Glycerincarbonat. Das Äquivalentverhältnis von Isocyanatgruppen zu Hydroxylgruppen beträgt 6.

Bei den heute bekannten, auf hydroxyfunktionellen cyclischen Carbonaten beruhenden Reaktivsystemen handelt es sich somit entweder um Umsetzungsprodukte derartiger Bausteine mit vergleichsweise niedermolekularen oligomeren Polyisocyanaten oder mit höhermolekularen Isocyanatprepolymeren, die in der Regel aber linear aufgebaut sind. Hochmolekulare und gleichzeitig hochfunktionelle Polyisocyanatkomponenten wurden als Reaktionspartner für hydroxyfunktionelle Cyclocarbonate bisher nicht beschrieben. Gerade polymere Polycyclocarbonatpolyurethane mit hohem Molekulargewicht sollten aber als Bindemittelkomponenten von besonderem Interesse sein, da sie z. B. in Kombination mit den kommerziell gut verfügbaren niedermolekularen Polyaminen, wie sie beispielsweise als Vernetzer für Epoxysysteme dienen, zu hochvernetzten besonders beständigen Polyurethanen abreagieren sollten.

Die Hauptursache für das bisherige Fehlen hochfunktioneller polymerer Polycyclocarbonatpolyurethane liegt darin, dass monomerarme NCO-Prepolymere hochmolekularer verzweigter Polyole aufgrund der während der Prepolymerisierungsreaktion mit Diisocyanaten unvermeidlich einsetzenden Vernetzungsreaktionen und dem damit verbundenen Molekulargewichtsaufbau gelieren oder zumindest extrem hohe Viskositäten aufweisen, die sie als Bausteine für eine Umsetzung mit hydroxyfunktionellen cyclischen Carbonaten unbrauchbar machen.

Aufgabe der vorliegenden Erfindung war es daher, ein Verfahren zur Herstellung von Aufbaukomponenten für die Synthese von Polyurethanen mit cyclischen Carbonatstrukturen, die als Bindemittel für Lacke und Beschichtungen geeignet sind, zur Verfügung zu stellen. Die nach dem erfindungsgemäßen Verfahren erhältlichen Aufbaukomponenten sollen die Herstellung von cyclische Carbonatstrukturen enthaltenden Polyurethanen aus beliebigen und somit auch beispielsweise hochverzweigten polymeren Polyolen sicher und reproduzierbar ermöglichen. Diese Aufgabe wird erfindungsgemäß gelöst durch ein Verfahren zur Herstellung Isocyanatgruppen und cyclische Carbonatstrukturen enthaltender Verbindungen, durch Umsetzung von wenigstens A) einem monomeren Diisocyanat mit aliphatisch, cycloaliphatisch, araliphatisch und/oder aromatisch gebundenen Isocyanatgruppen mit B) einem hydroxyfunktionellen cyclischen Carbonat, das dadurch gekennzeichnet ist, dass die Komponente A) mit Komponente B) in einem Äquivalentverhältnis von Isocyanatgruppen zu Hydroxylgruppen von mindestens 8 : 1 umgesetzt wird.

Das erfindungsgemäße Verfahren beruht auf der überraschenden Beobachtung, dass sich durch Reaktion üblicher hydroxyfunktioneller cyclischer Carbonate mit überschüssigen Mengen monomerer Diisocyanate in einem einem Äquivalentverhältnis von Isocyanatgruppen zu Hydroxylgruppen von mindestens 8 : 1, Verbindungen erhalten lassen, die gleichzeitig eine Isocyanatgruppe und eine cyclische Carbonatgruppe aufweisen und mit denen sich auch hochverzweigte polymere Polyole problemlos zu kristallisationsstabilen Polyurethanen mit Cyclocarbonat-Endgruppen umsetzen lassen. Die nicht umgesetzten monomeren Diisocyanate werden dabei vor der weiteren Umsetzung der Verbindungen abgetrennt.

Isocyanatgruppen und cyclische Carbonatstrukturen enthaltende Verbindungen sind grundsätzlich bereits bekannt. Beispielsweise beschreibt die EP-A 0 328 150 die Umsetzung von HDI mit Glycerincarbonat in organischer Lösung in einem Äquivalentverhältnis von Isocyanatgruppen zu Hydroxylgruppen von 6 : 1 mit anschließender destillativer Abtrennung des Lösungsmittels und des nicht umgesetzten überschüssigen Diisocyanats. Das dabei anfallende Reaktionsprodukt wurde analytisch jedoch nicht charakterisiert sondern unmittelbar zu einem cyclocarbonathaltigen Polyester weiterverarbeitet. Wie eigene Versuche zeigten, entsteht bei dem gewählten Äquivalentverhältnis allerdings ein sehr hoher Anteil des 2:1-Bisaddukts aus Glycerincarbonat und HDI, das eine starke Kristallisationstendenz aufweist. Der hohe Bisaddukt-Anteil, macht das nach EP 0 328 150 erhältliche Umsetzungsprodukt aus HDI und Glycerincarbonat als Baustein zur Herstellung trübungsstabiler Polycyclocarbonatpolyurethane für Lacke und Beschichtungen ungeeignet.

Gleiches gilt für die in der EP-A 0 703 230 beschriebenen 1,3-Dioxan-2-on-Gruppen enthaltenden Verbindungen aus Diisocyanaten und TMP-Carbonat, zu deren Herstellung auch ein geringer Diisocyanatüberschuß bis zu einem Äquivalentverhältnis von Isocyanatgruppen zu Hydroxylgruppen von 2 : 1 eingesetzt werden kann. Auch diese Verbindungen weisen einen sehr hohen Anteil des nicht kristallisationsstabilen 2:1-Bisaddukts aus hydroxyfunktionellem cyclischen Carbonat und Diisocyanat auf, weshalb sie als Aufbaukomponenten für Polycyclocarbonatpolyurethane ungeeignet sind.

Isocyanatgruppen und cyclische Carbonatstrukturen enthaltende Verbindungen, die unter Verwendung eines geringeren Diisocyanatüberschusses als erfindungsgemäß beansprucht hergestellt wurden und wie sie beispielsweise in der EP-A 0 703 230 und EP-A 0 328 150 beschrieben sind, liefern im Gegensatz zu den erfindungsgemäßen Verfahrensprodukten bei analoger Umsetzung mit Polyolen immer trübe Polycyclocarbonatpolyurethane, die als Bindemittel für Lacke und Beschichtungen unbrauchbar sind.

Ausgangskomponenten B) für das erfindungsgemäße Verfahren sind beliebige hydroxyfunktionelle cyclische Carbonate der allgemeinen Formel (I) oder deren Gemische, 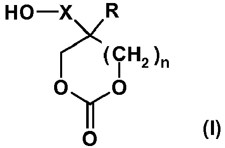 in welcher
- R: für Wasserstoff oder einen gesättigten oder ungesättigten, linearen oder verzweigten, aliphatischen Rest mit 1 bis 7 Kohlenstoffatomen steht,
- X: für einen linearen oder verzweigten organischen Rest mit 1 bis 36 Kohlenstoffatomen steht, der gegebenenfalls Ether-, Ester- und/oder Carbonatgruppen enthalten kann, und
- n: für 0 oder 1 steht.

Geeignete Ausgangskomponenten B) sind beispielsweise einfache hydroxyfunktionelle Cyclocarbonate, wie z. B. 4-(Hydroxymethyl)-1,3-dioxolan-2-on (Glycerincarbonat), 5-(Hydroxymethyl)-5-methyl-1,3-dioxan-2-on und/oder 5-(Hydroxymethyl)-5-ethyl-1,3-dioxan-2-on (TMP-Carbonat).

Geeignete Ausgangskomponenten B) sind daneben auch die durch Umsetzung dieser einfachen hydroxyfunktionellen cyclischen Carbonate mit Alkylenoxiden, wie z. B. Ethylenoxid und/oder Propylenoxid, Lactonen, wie z. B. β-Propiolacton, γ-Butyrolacton, δ-Valerolacton, ε-Caprolacton, 3,5,5- und 3,3,5-Trimethylcaprolacton, und/oder cyclischen Carbonaten, wie z. B. 1,3-Dioxan-2-on (Trimethylencarbonat) und 5,5-Dimethyl-1,3-dioxan-2-on (Neopentylglykolcarbonat), nach bekannten Methoden erhältlichen, cyclische Carbonatstrukturen aufweisenden Polyetheralkohole, Polyesteralkohole und/oder Polycarbonatalkohole mit zahlenmittleren Molekulargewichten von bis zu 600 g/mol, vorzugsweise bis zu 500 g/mol, besonders bevorzugt bis zu 400 g/mol.

Die Herstellung der Ausgangsverbindungen B) ist nicht Gegenstand der vorliegenden Anmeldung. Sie können nach bekannten Verfahren beispielsweise unter Verwendung fossiler Rohstoffe oder auch aus nachwachsenden Rohstoffen, wie z. B. aus biogenem Glycerin, wie es beispielsweise als Koppelprodukt der Biodieselherstellung anfällt, erhalten werden.

Als mögliche Herstellverfahren für die obengenannten einfachen hydroxyfunktionellen cyclischen Carbonate seien hier beispielhaft genannt Umsetzungen von Glycerin oder Trimethylolpropan mit Ethylencarbonat, Dialkyl- oder Diarylcarbonaten, wie z. B. Dimethyl- oder Diphenylcarbonat, unter Umesterungsbedingungen (siehe z. B. EP-A 1 963 301, EP-A 0 739 888, DE-A 196 25 265 und WO 2011/159219). Daneben kann Glycerincarbonat auch durch direkte Umsetzung von Glycidol mit Kohlendioxid in Gegenwart geeigneter Katalysatoren gewonnen werden (siehe z. B. EP-A 0 229 622).

Verfahren zur Herstellung der als Ausgangsverbindungen B) ebenfalls geeigneten Verbindungen, die zusätzlich mindestens eine Polyether-, Polyester- und/oder Polycarbonatgruppe enthalten sind ebenfalls bereits bekannt.

So liefert beispielsweise die Umsetzung einfacher hydroxyfunktioneller cyclischer Carbonate mit Alkylenoxiden, insbesondere mit Propylenoxid, nach den üblichen Verfahren zur Synthese von Polyethern (siehe z. B. N. Adam et al.: "Polyurethanes", Ullmann's Encyclopedia of Industrial Chemistry, Electronic Release, 7th ed., chap. 3.2.1, Wiley-VCH, Weinheim 2005) in Gegenwart von KOH oder insbesondere nach dem IMPACT-Prozess unter Verwendung von Doppelmetallcyanid-Katalysatoren (DMC) endständig cyclische Carbonatstrukturen aufweisende Polyetheralkohole, die als Ausgangsverbindung B) geeignet sind.

Bevorzugte mindestens eine Ethergruppe aufweisende Ausgangsverbindungen B) sind solche des obengenannten Molekulargewichtsbereiches auf Basis von Glycerincarbonat und Propylenoxid.

Geeignete Ausgangsverbindungen B), die neben einer cyclischen Carbonatgruppe mindestens eine Estergruppe enthalten, lassen sich in an sich bekannter Weise aus Lactonen und den oben beschriebenen einfachen hydroxyfunktionellen cyclischen Carbonaten als Startermolekülen unter Ringöffnung herstellen. Geeignete Lactone zur Herstellung dieser Estergruppen enthaltenden Ausgangsverbindungen B) sind beispielsweise β-Propiolacton, γ-Butyrolacton, δ-Valerolacton, ε-Caprolacton, 3,5,5- und 3,3,5-Trimethylcaprolacton oder beliebigen Gemische solcher Lactone.

Bevorzugte mindestens eine Estergruppe aufweisende Ausgangsverbindungen B) sind solche des obengenannten Molekulargewichtsbereiches auf Basis von Glycerincarbonat und ε-Caprolacton. Als Ausgangsverbindungen B) ebenfalls geeignet sind schließlich auch Verbindungen, die neben einer cyclischen Carbonatgruppe mindestens eine weitere Carbonatgruppe enthalten. Derartige Verbindungen sind ebenfalls bereits bekannt und beispielsweise nach dem in Beispiel 1), Step (A) der WO 03/016298 beschriebenen Verfahren durch Umsetzung der oben beschriebenen einfachen hydroxyfunktionellen cyclischen Carbonate mit Trimethylencarbonat und/oder Neopentylglykolcarbonat erhältlich.

Bevorzugte mindestens eine weitere Carbonatgruppe aufweisende Ausgangsverbindungen B) sind solche des obengenannten Molekulargewichtsbereiches auf Basis von Glycerincarbonat und Neopentylglykolcarbonat.

Im Allgemeinen erfolgt die Herstellung der vorstehend beschriebenen, für das erfindungsgemäße Verfahren geeigneten Ausgangsvebindungen B), die neben einer cyclischen Carbonatgruppe mindestens eine Estergruppe und/oder mindestens eine weitere Carbonatgruppe enthalten, durch ringöffnende Polymerisation in Gegenwart von Katalysatoren wie beispielsweise Lewis- oder Brönstedt-Säuren, organischen Zinn- oder Titanverbindungen bei Temperaturen von 20 bis 200°C, vorzugsweise 50 bis 160°C.

Für das erfindungsgemäße Verfahren bevorzugte Ausgangskomponenten B) sind hydroxyfunktionelle cyclische Carbonate der allgemeinen Formel (I) oder deren Gemische, in welcher
- R: für Wasserstoff oder einen gesättigten linearen aliphatischen Rest mit 1 oder 2 Kohlenstoffatomen steht,
- X: für einen linearen oder verzweigten organischen Rest mit 1 bis 18 Kohlenstoffatomen steht, der gegebenenfalls Ether-, Ester- und/oder Carbonatgruppen enthalten kann, und
- n: für 0 oder 1 steht.

Besonders bevorzugte Ausgangskomponenten B) sind hydroxyfunktionelle cyclische Carbonate der allgemeinen Formel (I) oder deren Gemische, in welcher
- R: für Wasserstoff oder einen gesättigten linearen aliphatischen Rest mit 1 oder 2 Kohlenstoffatomen steht,
- X: für eine Methylengruppe (-CH₂-) und
- n: für 0 oder 1 steht.

Ganz besonders bevorzugte Ausgangskomponente B) ist Glycerincarbonat.

Die hydroxyfunktionellen cyclischen Carbonate B) können beim erfindungsgemäßen Verfahren sowohl einzeln als auch in Form beliebiger Gemische untereinander eingesetzt werden.

Als Ausgangsverbindungen A) für das erfindungsgemäße Verfahren sind beliebige Diisocyanate mit aliphatisch, cycloaliphatisch, araliphatisch und/oder aromatisch gebundenen Isocyanatgruppen geeignet, die nach beliebigen Verfahren, z. B. durch Phosgenierung oder auf phosgenfreiem Weg, beispielsweise durch Urethanspaltung, hergestellt werden können.

Bevorzugte Diisocyanate A) sind solche der allgemeinen Formel (II)

**OCN-Y-NCO** (II),

in welcher Y für einen linearen oder verzweigten, aliphatischen oder cycloaliphatischen Rest mit 4 bis 18 Kohlenstoffatomen oder einen gegebenenfalls substituierten aromatischen oder araliphatischen Rest mit 6 bis 18 Kohlenstoffatomen steht.

Geeignet sind beispielsweise z. B. 1,4-Diisocyanatobutan, 1,6-Diisocyanatohexan (HDI), 1,5-Diisocyanato-2,2-dimethylpentan, 2,2,4- bzw. 2,4,4-Trimethyl-1,6-diisocyanatohexan, 1,10-Diisocyanatodecan, 1,3- und 1,4-Diisocyanatocyclohexan, 1,4-Diisocyanato-3,3,5-trimethylcyclohexan, 1,3-Diisocyanato-2-methylcyclohexan, 1,3-Diisocyanato-4-methylcyclohexan, 1-Isocyanato-3,3,5-trimethyl-5-isocyanatomethylcyclohexan (Isophorondiisocyanat; IPDI), 1-Isocyanato-1-methyl-4(3)-isocyanatomethylcyclohexan, 2,4'- und 4,4'-Diisocyanatodicyclohexylmethan (H₁₂-MDI), 1,3- und 1,4-Bis(isocyanatomethyl)cyclohexan, 4,4'-Diisocyanato-3,3'-dimethyl-dicyclohexylmethan, 4,4'-Diisocyanato-3,3',5,5'-tetramethyldicyclohexylmethan, 4,4'-Diisocyanato-1,1'-bi(cyclohexyl), 4,4'-Diisocyanato-3,3'-dimethyl-1,1'-bi(cyclohexyl), 4,4'-Diisocyanato-2,2',5,5'-tetra-methyl-1,1'-bi(cyclohexyl), 1,8-Diisocyanato-p-menthan, 1,3-Diisocyanatoadamantan, 1,3-Dimethyl-5,7-diisocyanatoadamantan, 1,3- und 1,4-Bis-(isocyanatomethyl)benzol (XDI), 1,3- und 1,4-Bis(1-isocyanato-1-methylethyl)-benzol (TMXDI), Bis(4-(1-isocyanato-1-methylethyl)phenyl)-carbonat, 1,3- und 1,4-Phenylendiisocyanat, 2,4- und 2,6-Toluylendiisocyanat (TDI) sowie beliebige Gemische dieser Isomeren, Diphenylmethan-2,4'- und/oder -4,4'-diisocyanat (MDI) und Naphthylen-1,5-diisocyanat (NDI) sowie beliebige Gemische solcher Diisocyanate. Weitere ebenfalls geeignete Diisocyanate finden sich darüberhinaus beispielsweise in Justus Liebigs Annalen der Chemie Band 562 (1949) S. 75 - 136.

Als Ausgangskomponente A) besonders bevorzugt sind Diisocyanate der allgemeinen Formel (II), in welcher Y für einen linearen oder verzweigten, aliphatischen oder cycloaliphatischen Rest mit 6 bis 13 Kohlenstoffatomen steht.

Besonders bevorzugte Ausgangskomponenten A) für das erfindungsgemäße Verfahren sind 1,6-Diisocyanatohexan, 1-Isocyanato-3,3,5-trimethyl-5-isocyanatomethylcyclohexan, 2,4'- und/oder 4,4'-Diisocyanatodicyclohexylmethan oder deren Gemische.

In einer bevorzugten Ausführungsform des erfindungsgemäßen Verfahrens werden die Diisocyanate A) mit mindestens einem hydroxyfunktionellen cyclischen Carbonat B) bei Temperaturen von 20 bis 200 °C, vorzugsweise 40 bis 160 °C umgesetzt.

Dabei wird Komponente A) mit Komponente B) in einem Äquivalentverhältnis von Isocyanatgruppen zu Hydroxylgruppen von mindestens 8 : 1, bevorzugt mindestens 10 : 1 und besonders bevorzugt mindestens 12 : 1 umgesetzt.

In einer weiteren bevorzugten Ausführungsform des erfindungsgemäßen Verfahrens wird die Komponente A) mit Komponente B) in einem Äquivalentverhältnis von Isocyanatgruppen zu Hydroxylgruppen von höchstens 40 : 1, bevorzugt von höchstens 30 : 1 umgesetzt.

Die Umsetzung der Ausgangskomponenten A) und B) kann nach dem erfindungsgemäßen Verfahren in Lösung oder lösemittelfrei in Substanz, bevorzugt jedoch lösemittelfrei ausgeführt werden.

Die Reaktion kann unkatalysiert durchgeführt werden. Gegebenenfalls können zur Beschleunigung der Umsetzung aber auch übliche aus der Polyurethanchemie bekannte Katalysatoren mitverwendet werden. Beispielhaft seien hier genannt tert. Amine, wie z. B. Triethylamin, Tributylamin, Dimethylbenzylamin, Diethylbenzylamin, Pyridin, Methylpyridin, Dicyclohexylmethylamin, Dimethylcyclohexylamin, N,N,N',N'-Tetramethyldiaminodiethylether, Bis-(dimethylaminopropyl)-harnstoff, N-Methyl- bzw. N-Ethylmorpholin, N-Cocomorpholin, N-Cyclohexylmorpholin, N,N,N',N'-Tetramethylethylendiamin, N,N,N',N'-Tetramethyl-1,3-butandiamin, N,N,N',N'-Tetramethyl-1,6-hexandiamin, Pentamethyldiethylentriamin, N-Methylpiperidin, N-Dimethylaminoethylpiperidin, N,N'-Dimethylpiperazin, N-Methyl-N'-dimethylaminopiperazin, 1,2-Dimethylimidazol, 2-Methylimidazol, N,N-Dimethylimidazol-ß-phenylethylamin, 1,4-Diazabicyclo-(2,2,2)-octan (DABCO) und Bis-(N,N-dimethylaminoethyl)adipat, Amidine, wie z. B. 1,5-Diazabicyclo[4.3.0]nonen (DBN), 1,8-Diazabicyclo(5.4.0)undecen-7 (DBU) und 2,3-Dimethyl-3,4,5,6-tetrahydropyrimidin, Alkanolaminverbindungen, wie z. B. Triethanolamin, Triisopropanolamin, N-Methyl-diethanolamin, N-Ethyl-diethanolamin, Dimethylaminoethanol und 2-(N,N-Dimethylaminoethoxy)ethanol, N,N',N"-Tris-(dialkylaminoalkyl)hexahydrotriazine, wie z. B. N,N',N"-Tris-(dimethylaminopropyl)-s-hexahydrotriazin, Bis(dimethylaminoethyl)ether sowie Metallsalze, wie z. B. anorganische und/oder organische Verbindungen des Eisens, Bleis, Wismuths, Zinks, und/oder Zinns in üblichen Oxidationsstufen des Metalls, beispielsweise Eisen(II)-chlorid, Eisen(III)-chlorid, Wismut(III)- Wismut(III)-2-ethylhexanoat, Wis-mut(III)-octoat, Wismut(III)-neodecanoat, Zinkchlorid, Zink-2-ethylcaproat, Zinn(II)-octoat, Zinn(II)-ethylcaproat, Zinn(II)-palmitat, Dibutylzinn(IV)-dilaurat (DBTL), Dibutylzinn(IV)-dichlorid oder Bleioctoat.

Bevorzugt einzusetzende Katalysatoren sind tertiäre Amine, Zinn-, Zink und Wismutverbindungen der genannten Art.

Die beispielhaft genannten Katalysatoren können bei der Herstellung der erfindungsgemäßen Isocyanatgruppen und cyclische Carbonatstrukturen enthaltenden Verbindungen einzeln oder in Form beliebiger Mischungen untereinander eingesetzt werden und kommen dabei, falls überhaupt, in Mengen von 0,001 bis 1,0 Gew.-%, bevorzugt 0,005 bis 0,5 Gew.-%, berechnet als Gesamtmenge an eingesetzten Katalysatoren bezogen auf die Gesamtmenge der verwendeten Ausgangsverbindungen, zum Einsatz.

Der Verlauf der Umsetzung kann beim erfindungsgemäßen Verfahren durch z. B. titrimetrische Bestimmung des NCO-Gehaltes verfolgt werden. Nach Erreichen des angestrebten NCO-Gehaltes, im allgemeinen nach vollständiger Urethanisierung, wird die Reaktion abgebrochen.

In einer bevorzugten Ausführungsform der Erfindung wird nach der Umsetzung der Komponenten A) und B) ein nicht umgesetzter Überschusses an monomeren Diisocyanaten A) bis auf einen Restgehalt von weniger als 1 Gew.-%, vorzugsweise weniger als 0,5 Gew.-%, besonders bevorzugt von weniger als 0,3 Gew.-%, bezogen auf die Gesamtmasse des Reaktionsproduktes, vom Reaktionsprodukt abgetrennt.

Das Reaktionsgemisch wird dabei vorzugsweise durch Dünnschichtdestillation im Vakuum, beispielsweise bei einem Druck von unter 1,0 mbar, vorzugsweise unter 0,5 mbar, besonders bevorzugt unter 0,2 mbar, unter möglichst schonenden Bedingungen, beispielsweise bei einer Temperatur von 100 bis 200 °C, vorzugsweise von 120 bis 180 °C, von überschüssigen monomeren Diisocyanaten befreit.

Die anfallenden Destillate können problemlos zur erneuten Umsetzung mit geeigneten Ausgangsverbindungen B) verwendet werden.

In einer weiteren, jedoch weniger bevorzugten Ausführungsform des erfindungsgemäßen Verfahrens werden die monomeren Diisocyanate durch Extraktion mit geeigneten gegenüber Isocyanatgruppen inerten Lösungsmitteln, beispielsweise aliphatischen oder cycloaliphatischen Kohlenwasserstoffen wie Pentan, Hexan, Heptan, Cyclopentan oder Cyclohexan vom gebildeten erfindungsgemäßen Verfahrensprodukt abgetrennt.

Unabhängig von der Art der Aufarbeitung erhält man als Produkte des erfindungsgemäßen Verfahrens klare, praktisch farblose Isocyanatgruppen und cyclische Carbonatstrukturen enthaltende Verbindungen, die in Abhängigkeit vom gewählten Ausgangsdiisocyanat niedrig- bis hochviskose Flüssigkeiten oder feste Stoffe darstellen und NCO-Gehalte von 4,5 bis 14,8 Gew.-%, vorzugsweise 5,0 bis 14,8 Gew.-%, besonders bevorzugt 9,0 bis 14,5 Gew.-% sowie Restgehalte an monomeren Ausgangsdiisocyanaten von weniger als 1,0 Gew.-%, vorzugsweise von weniger als 0,5 Gew.-%, besonders bevorzugt von weniger als 0,3 Gew.-%, bezogen auf die Gesamtmasse des Reaktionsproduktes, aufweisen.

Ein weiterer Gegenstand der Erfindung sind Isocyanatgruppen und cyclische Carbonatstukturen enthaltende Verbindungen erhältlich nach dem erfindungsgemäßen Verfahren.

Ebenfalls Gegenstand der Erfindung ist eine Zusammensetzung, enthaltend
a) Isocyanatgruppen und cyclische Carbonatstukturen enthaltende Verbindungen der allgemeinen Formel (III), 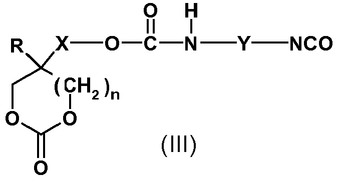
b) zwei cyclische Carbonatstukturen enthaltende Verbindungen der allgemeinen Formel (IV) 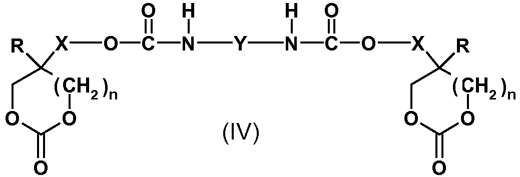 und
c) monomere Diisocyanate mit aliphatisch, cycloaliphatisch, araliphatisch und/oder aromatisch gebundenen Isocyanatgruppen,
wobei in den Formeln (III) und (IV)
- R: für Wasserstoff oder einen gesättigten oder ungesättigten, linearen oder verzweigten, aliphatischen Rest mit 1 bis 7 Kohlenstoffatomen steht,
- X: für einen linearen oder verzweigten organischen Rest mit 1 bis 36 Kohlenstoffatomen steht, der gegebenenfalls Ether-, Ester- und/oder Carbonatgruppen enthalten kann,
- n: für 0 oder 1 steht und
- Y: für einen linearen oder verzweigten, aliphatischen oder cycloaliphatischen Rest mit 4 bis 18 Kohlenstoffatomen oder einen gegebenenfalls substituierten aromatischen oder araliphatischen Rest mit 6 bis 18 Kohlenstoffatomen steht,
dadurch gekennzeichnet, dass jeweils bezogen auf die Gesamtmenge der Komponenten a) und b) die Komponente a) einen Anteil von ≥ 88 Gew.-%, bevorzugt ≥ 90 Gew.-%, besonders bevorzugt ≥ 92 Gew.-%, und die Komponente b) einen Anteil von ≤ 12 Gew.-%, bevorzugt ≤ 10 Gew.-%, besonders bevorzugt ≤ 8 Gew.-%, ausmacht und die Komponente c) zu ≤ 1 Gew.-% in der Gesamtzusammensetzung enthalten ist.

In einer bevorzugten Ausführungsform der Erfindung macht die Komponente b) einen Anteil von 0,5 bis 12 Gew.-%, bevorzugt 0,5 bis 10 Gew.-%, besonders bevorzugt von 0,5 bis 8 Gew.-% bezogen auf die Gesamtmenge der Komponenten a) und b) aus.

Bevorzugte erfindungsgemäße Zusammensetzungen sind dabei solche, bei denen in den Formeln (III) und (IV)
- R: für Wasserstoff oder einen gesättigten linearen aliphatischen Rest mit 1 oder 2 Kohlenstoffatomen,
- X: für einen linearen oder verzweigten organischen Rest mit 1 bis 18 Kohlenstoffatomen, der gegebenenfalls Ether-, Ester- und/oder Carbonatgruppen enthalten kann, und
- n: für 0 oder 1 steht.

Besonders bevorzugte erfindungsgemäße Zusammensetzungen sind solche, bei denen in den Formeln (III) und (IV)
- R: für Wasserstoff oder einen gesättigten linearen aliphatischen Rest mit 1 oder 2 Kohlenstoffatomen,
- X: für eine Methylengruppe (-CH₂-) und
- n: für 0 oder 1.

Ebenfalls bevorzugte erfindungsgemäße Zusammensetzungen sind solche, bei denen in den Formeln (III) und (IV)
- Y: für einen linearen oder verzweigten, aliphatischen oder cycloaliphatischen Rest mit 6 bis 13 Kohlenstoffatomen steht.

Für die monomeren Diisocyanate c) gelten ebenfalls die für die Ausgangskomponente A) oben angegebenen bevorzugten Ausführungsformen.

Gegenstand der Erfindung ist insbesondere auch die Verwendung der erfindungsgemäßen Isocyanatgruppen und cyclische Carbonatstrukturen enthaltenden Verbindungen oder der erfindungsgemäßen Zusammensetzung von Isocyanatgruppen und cyclische Carbonatstukturen enthaltenden Verbindungen als Ausgangskomponenten bei der Herstellung von cyclische Carbonatstrukturen enthaltenden Polyurethanen, vernetzbaren Bindemitteln, sowie vernetzbaren Lack-, Dichtstoff- oder Klebstoffrohstoffen. Die cyclische Carbonatstrukturen enthaltenden Polyurethanen oder vernetzbaren Bindemittel werden dabei bevorzugt zur Herstellung von Lack-, Dichtstoff- oder Klebstoffrohstoffen verwendet.

Ein weiterer Gegenstand der Erfindung sind cyclische Carbonatstrukturen enthaltende Polyurethane, hergestellt unter Verwendung der erfindungsgemäßen Isocyanatgruppen und cyclische Carbonatstrukturen enthaltenden Verbindungen oder die erfindungsgemäße Zusammensetzung von Isocyanatgruppen und cyclische Carbonatstukturen enthaltenden Verbindungen.

Diese können durch Umsetzung der erfindungsgemäßen Isocyanatgruppen und cyclische Carbonatstrukturen enthaltenden Verbindungen oder der erfindungsgemäße Zusammensetzung von Isocyanatgruppen und cyclische Carbonatstrukturen enthaltenden Verbindungen mit beliebigen vorzugsweise mindestens difunktionellen Polyolen, wie z. B. einfachen mehrwertigen Alkoholen, Ether- oder Esteralkoholen, oder üblichen aus der Polyurethanchemie bekannten polymeren Polyetherpolyolen, Polyesterpolyolen, Polycarbonatpolyolen und/oder Polyacrylatpolyolen, hergestellt werden.

### Beispiele

Die Erfindung wird im Folgenden an Hand von Beispielen näher erläutert.

Alle Prozentangaben beziehen sich, soweit nichts Anderslautendes vermerkt, auf das Gewicht.

Die Bestimmung der NCO-Gehalte erfolgte titrimetrisch nach DIN EN ISO 11909.

OH-Zahlen wurden titrimetrisch nach DIN 53240-2: 2007-11 bestimmt.

Die Rest-Monomeren Gehalte wurden gaschromatographisch nach DIN EN ISO 10283 gemessen.

Die Anteile an Bisaddukt (aus zwei Molekülen hydroxyfunktionellem cyclischen Carbonat und einem Molekül Diisocyanat) wurden durch Gelpermationschromatographie in Anlehnung an DIN 55672-1 (Gelpermeationschromatographie (GPC) - Teil 1: Tetrahydrofuran (THF) als Elutionsmittel) bei Raumtemperatur ermittelt, mit der Änderung, dass mit einer Flußrate von 0,6 ml/min statt 1,0 ml/min gearbeitet wurde. Die den Chromatogrammen entnommenen Anteile an Bisaddukt in Flächen-%, welche softwaregestützt ermittelt wurden, wurden näherungsweise jeweils Anteilen in Gew.-% gleichgesetzt und als solche, bezogen auf die Gesamtmenge an Mono- und Bisaddukt, angegeben.

Sämtliche Viskositätsmessungen erfolgten mit einem Physica MCR 51 Rheometer der Fa. Anton Paar Germany GmbH (DE) nach DIN EN ISO 3219.

Die angegebenen Schmelzbereiche wurden nach ANSI / ASTM D 3451-76 mit Hilfe einer Kofler-Heizbank der Fa. Wagner & Munz GmbH (DE) ermittelt.

### Beispiel 1 (erfindungsgemäß)

1344 g (8 mol) Hexamethylendiisocyanat (HDI) wurden bei einer Temperatur von 100°C unter trockenem Stickstoff vorgelegt, innerhalb von 30 Minuten mit 118 g (1 mol) Glycerincarbonat versetzt und weitere 5 Stunden gerührt, bis ein NCO-Gehalt von 43,1 %, entsprechend einer vollständigen Urethanisierung, erreicht war. Anschließend wurde das nicht umgesetzte monomere HDI bei einer Temperatur von 140°C und einem Druck von 0,1 mbar im Dünnschichtverdampfer abgetrennt. Man erhielt ein praktisch farbloses, klares isocyanatfunktionelles cyclisches Carbonat, das bei Raumtemperatur innerhalb einer Woche langsam durchkristallisierte.

Das Produkt wies die folgenden Kenndaten auf:

| | |
|---|---|
| NCO-Gehalt: | 14,0 % |
| monomeres HDI: | 0,18 % |
| Schmelzbereich: | 28 - 30°C |
| Anteil Bisaddukt: | 4,6 % |

### Beispiel 2 (erfindungsgemäß)

Nach dem in Beispiel 1 beschriebenen Verfahren wurden 1008 g (6 mol) HDI mit 118 g (1 mol) Glycerincarbonat umgesetzt. Nach Erreichen eines NCO-Gehalts von 41,0 %, entsprechend einer vollständigen Urethanisierung, wurde das nicht umgesetzte monomere HDI wie in Beispiel 1 beschrieben durch Dünnschichtdestillation entfernt und man erhielt ein praktisch farbloses, klares isocyanatfunktionelles cyclisches Carbonat, das bei Raumtemperatur innerhalb einer Woche langsam durchkristallisierte.

Das Produkt wies die folgenden Kenndaten auf:

| | |
|---|---|
| NCO-Gehalt: | 14,0 % |
| monomeres HDI: | 0,18 % |
| Schmelzbereich: | 28 - 30°C |
| Anteil Bisaddukt: | 6,1 % |

### Beispiel 3 (erfindungsgemäß)

Nach dem in Beispiel 1 beschriebenen Verfahren wurden 1680 g (10 mol) HDI mit 118 g (1 mol) Glycerincarbonat umgesetzt. Nach Erreichen eines NCO-Gehalts von 44,4 %, entsprechend einer vollständigen Urethanisierung, wurde das nicht umgesetzte monomere HDI wie in Beispiel 1 beschrieben durch Dünnschichtdestillation entfernt und man erhielt ein praktisch farbloses, klares isocyanatfunktionelles cyclisches Carbonat, das bei Raumtemperatur innerhalb einer Woche langsam durchkristallisierte.

Das Produkt wies die folgenden Kenndaten auf:

| | |
|---|---|
| NCO-Gehalt: | 14,1 % |
| monomeres HDI: | 0,19 % |
| Schmelzbereich: | 28 - 30°C |
| Anteil Bisaddukt: | 3,8 % |

### Beispiel 4 (erfindungsgemäß)

Nach dem in Beispiel 1 beschriebenen Verfahren wurden 1680 g (10 mol) HDI mit 59 g (0,5 mol) Glycerincarbonat umgesetzt. Nach Erreichen eines NCO-Gehalts von 45,9 %, entsprechend einer vollständigen Urethanisierung, wurde das nicht umgesetzte monomere HDI wie in Beispiel 1 beschrieben durch Dünnschichtdestillation entfernt und man erhielt ein praktisch farbloses, klares isocyanatfunktionelles cyclisches Carbonat, das bei Raumtemperatur innerhalb einer Woche langsam durchkristallisierte.

Das Produkt wies die folgenden Kenndaten auf:

| | |
|---|---|
| NCO-Gehalt: | 14,4 % |
| monomeres HDI: | 0,09 % |
| Schmelzbereich: | 29 - 31°C |
| Anteil Bisaddukt: | 1,9 % |

### Beispiel 5 (erfindungsgemäß)

Nach dem in Beispiel 1 beschriebenen Verfahren wurden 672 g (4 mol) HDI mit 118 g (1 mol) Glycerincarbonat umgesetzt. Nach Erreichen eines NCO-Gehalts von 37,2 %, entsprechend einer vollständigen Urethanisierung, wurde das nicht umgesetzte monomere HDI wie in Beispiel 1 beschrieben durch Dünnschichtdestillation entfernt und man erhielt ein praktisch farbloses, klares isocyanatfunktionelles cyclisches Carbonat, das bei Raumtemperatur innerhalb einer Woche langsam durchkristallisierte.

Das Produkt wies die folgenden Kenndaten auf:

| | |
|---|---|
| NCO-Gehalt: | 13,4 % |
| monomeres HDI: | 0,11 % |
| Schmelzbereich: | 27 - 30°C |
| Anteil Bisaddukt: | 9,1 % |

### Beispiel 6 (erfindungsgemäß)

Nach dem in Beispiel 1 beschriebenen Verfahren wurden 1776 g (8 mol) Isophorondiisocyanat (IPDI) mit 118 g (1 mol) Glycerincarbonat umgesetzt. Nach Erreichen eines NCO-Gehalts von 33,3 %, entsprechend einer vollständigen Urethanisierung, wurde das nicht umgesetzte monomere IPDI bei einer Temperatur von 160°C und einem Druck von 0,2 mbar durch Dünnschichtdestillation entfernt und man erhielt ein isocyanatfunktionelles cyclisches Carbonat in Form eines schwach gelb gefärbten, klaren Festharzes.

| | |
|---|---|
| NCO-Gehalt: | 11,8 % |
| monomeres IPDI: | 0,21 % |
| Anteil Bisaddukt: | 4,5 % |

### Beispiel 7 (erfindungsgemäß)

Nach dem in Beispiel 1 beschriebenen Verfahren wurden 2096 g (8 mol) 4,4'-Diisocyanatodicyclohexylmethan (H₁₂-MDI) mit 118 g (1 mol) Glycerincarbonat umgesetzt. Nach Erreichen eines NCO-Gehalts von 28,5 %, entsprechend einer vollständigen Urethanisierung, wurde das nicht umgesetzte monomere H₁₂-MDI bei einer Temperatur von 170°C und einem Druck von 0,2 mbar durch Dünnschichtdestillation entfernt und man erhielt ein isocyanatfunktionelles cyclisches Carbonat in Form eines schwach gelb gefärbten, klaren Festharzes.

| | |
|---|---|
| NCO-Gehalt: | 10,3 % |
| monomeres H₁₂-MDI: | 0,28 % |
| Anteil Bisaddukt: | 4,6 % |

### Beispiel 8 (erfindungsgemäß)

Nach dem in Beispiel 1 beschriebenen Verfahren wurden 1344 g (8 mol) HDI mit 160 g (1 mol) TMP-Carbonat umgesetzt. Nach Erreichen eines NCO-Gehalts von 41,9 %, entsprechend einer vollständigen Urethanisierung, wurde das nicht umgesetzte monomere HDI wie in Beispiel 1 beschrieben durch Dünnschichtdestillation entfernt und man erhielt ein farbloses, klares isocyanatfunktionelles cyclisches Carbonat mit folgenden Kenndaten:

| | |
|---|---|
| NCO-Gehalt: | 12,1 % |
| monomeres HDI: | 0,17 % |
| Viskosität (23°C): | 77000 mPas |
| Anteil Bisaddukt: | 5,1 % |

### Beispiel 9 (erfindungsgemäß)

118 g (1 mol) Glycerincarbonat und 114 g (1 mol) ε-Caprolacton wurden bei Raumtemperatur unter trockenem Stickstoff vermischt, mit 0,02 g ortho-Phosphorsäure versetzt und anschließend für 5 h auf 160 °C erhitzt. Nach Abkühlen auf Raumtemperatur lag ein farbloser endständig cyclische Carbonatstrukturen aufweisender Polyesteralkohol mit folgenden Kenndaten vor:

| | |
|---|---|
| OH-Zahl: | 244 mg KOH/g |
| freies ε-Caprolacton: | 0,15 % |
| Viskosität (23°C): | 2950 mPas |
| mittleres Molekulargewicht (aus OH-Zahl ber.): | 230 |

230 g (1,0 mol) dieses Glycerincarbonat/ε-Caprolacton-Adduktes wurden nach dem in Beispiel 1 beschriebenen Verfahren mit 1344 g (8 mol) HDI umgesetzt. Nach Erreichen eines NCO-Gehalts von 40,0 %, entsprechend einer vollständigen Urethanisierung, wurde das nicht umgesetzte monomere HDI wie in Beispiel 1 beschrieben durch Dünnschichtdestillation entfernt und man erhielt ein praktisch farbloses, klares isocyanatfunktionelles, Estergruppen aufweisendes cyclisches Carbonat mit folgenden Kenndaten:

| | |
|---|---|
| NCO-Gehalt: | 9,8 % |
| monomeres HDI: | 0,19 % |
| Viskosität (23°C): | 3100 mPas |
| Anteil Bisaddukt: | 5,3 % |

### Beispiel 10 (Vergleich)

Nach dem in Beispiel 1 beschriebenen Verfahren wurden 504 g (3 mol) HDI mit 118 g (1 mol) Glycerincarbonat umgesetzt. Nach Erreichen eines NCO-Gehalts von 33,8 %, entsprechend einer vollständigen Urethanisierung, wurde das nicht umgesetzte monomere HDI wie in Beispiel 1 beschrieben durch Dünnschichtdestillation entfernt und man erhielt ein praktisch farbloses, klares isocyanatfunktionelles cyclisches Carbonat, das nach Abkühlung auf Raumtemperatur teilweise kristallisierte.

Das Produkt wies die folgenden Kenndaten auf:

| | |
|---|---|
| NCO-Gehalt: | 12,7 % |
| monomeres HDI: | 0,19 % |
| Anteil Bisaddukt: | 13,5 % |

### Beispiel 11 (Verwendung, erfindungsgemäß)

270 g (0,9 mol) des isocyanatfunktionellen cyclischen Carbonats aus Beispiel 1 wurden unter trockenem Stickstoff in 207 g Tetramethoxyethan als Lösungsmittel bei einer Temperatur von 80°C vorgelegt, innerhalb von 20 min portionsweise mit 40 g (0,3 mol) Trimethylolpropan (TMP) versetzt und weitere 4 Stunden bis zum vollständigen Verschwinden der Isocyanatbande im IR-Spektrum gerührt. Die vorliegende 60 %ige klare Lösung eines endständig Cyclocarbonatgruppen tragenden Polyurethans wies bei 23°C eine Viskosität von 5.540 mPas auf.

### Beispiel 12 (Vergleich)

1680 g (10 mol) HDI wurden bei einer Temperatur von 80°C unter trockenem Stickstoff innerhalb einer Stunde mit 134 g (1 mol) TMP versetzt und eine weitere Stunde gerührt, bis ein NCO-Gehalt von 39,4 %, entsprechend einer vollständigen Urethanisierung, erreicht war. Anschließend wurde das nicht umgesetzte monomere HDI bei einer Temperatur von 130°C und einem Druck von 0,1 mbar im Dünnschichtverdampfer abgetrennt. Man erhielt ein farbloses klares Polyisocyanat mit einem NCO-Gehalt von 16,7 %, einem Gehalt an monomerem HDI von 0,21 % und einer Viskosität (23°C) von 480.000 mPas.

251 g (1,0 val) dieses hochviskosen Polyisocyanats wurden unter trockenem Stickstoff in 246 g Tetramethoxyethan gelöst, anschließend bei einer Temperatur von 80°C mit 118 g (1,0 mol) Glycerincarbonat versetzt und weitere 5 Stunden bis zum vollständigen Verschwinden der Isocyanatbande im IR-Spektrum gerührt. Die vorliegende 60 %ige Lösung eines endständig Cyclocarbonatgruppen tragenden Polyurethans wies bei 23°C eine Viskosität von 12.360 mPas auf.

Im Vergleich zu dem nach Beispiel 11 erhaltenen Umsetzungsprodukt eines erfindungsgemäßen isocyanatfunktionellen cyclischen Carbonats mit TMP zeigt das nach Beispiel 12 hergestellte Umsetzungsprodukt eines hydroxyfunktionellen cyclischen Carbonats mit einem monomerenarmen Isocyanatprepolymer auf Basis von TMP eine erheblich niedrigere Viskosität obwohl beide Polyurethane auf den gleichen Ausgangskomponenten beruhen.

### Beispiel 13 (Verwendung, erfindungsgemäß)

282 g (0,9 mol) des isocyanatfunktionellen cyclischen Carbonats aus Beispiel 5 wurden unter trockenem Stickstoff in 215 g Tetramethoxyethan als Lösungsmittel bei einer Temperatur von 80°C vorgelegt, innerhalb von 20 min portionsweise mit 40 g (0,3 mol) Trimethylolpropan (TMP) versetzt und weitere 4 Stunden bis zum vollständigen Verschwinden der Isocyanatbande im IR-Spektrum gerührt. Die vorliegende 60 %ige klare Lösung eines endständig Cyclocarbonatgruppen tragenden Polyurethans wies bei 23°C eine Viskosität von 5.220 mPas auf.

### Beispiel 14 (Verwendung, Vergleich)

298 g (0,9 mol) des nicht erfindungsgemäßen isocyanatfunktionellen cyclischen Carbonats aus Beispiel 10 wurden unter trockenem Stickstoff in 225 g Tetramethoxyethan als Lösungsmittel bei einer Temperatur von 80°C vorgelegt, innerhalb von 20 min portionsweise mit 40 g (0,3 mol) Trimethylolpropan (TMP) versetzt und weitere 4 Stunden bis zum vollständigen Verschwinden der Isocyanatbande im IR-Spektrum gerührt. Die vorliegende 60 %ige Lösung eines endständig Cyclocarbonatgruppen tragenden Polyurethans trübte unmittelbar nach Abkühlen auf Raumtemperatur stark ein und bildete innerhalb eines Tages einen deutlichen Bodensatz.

Der Vergleich mit dem nach Beispiel 13) als klare Lösung erhaltenen Umsetzungsprodukt des erfindungsgemäßen isocyanatfunktionellen cyclischen Carbonats aus Beispiel 1) mit TMP zeigt, dass das isocyanatfunktionelle cyclische Carbonat aus Beispiel 10), das unter Verwendung eines geringeren als dem erfindungsgemäßen Mindestüberschuss an Isocyanatgruppen hergestellt wurde, aufgrund eines zu hohen Anteils an Bisaddukt nicht kristallisationsstabil und somit zur Herstellung Cyclocarbonatgruppen tragender Polyurethane ungeeignet ist.

## Patentansprüche

1. Verfahren zur Herstellung Isocyanatgruppen und cyclische Carbonatstrukturen enthaltender Verbindungen, durch Umsetzung von wenigstens
A) einem monomeren Diisocyanat mit aliphatisch, cycloaliphatisch, araliphatisch und/oder aromatisch gebundenen Isocyanatgruppen
mit
B) einem hydroxyfunktionellen cyclischen Carbonat,
**dadurch gekennzeichnet, dass** die Komponente A) mit Komponente B) in einem Äquivalentverhältnis von Isocyanatgruppen zu Hydroxylgruppen von mindestens 8 : 1 umgesetzt wird.

2. Verfahren gemäß Anspruch 1, **dadurch gekennzeichnet, dass** als Komponente B) hydroxyfunktionelle cyclische Carbonate der allgemeine Formel (I) oder deren Gemische eingesetzt werden, 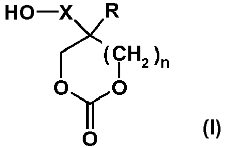 wobei
R für Wasserstoff oder einen gesättigten oder ungesättigten, linearen oder verzweigten, aliphatischen Rest mit 1 bis 7 Kohlenstoffatomen steht,
X für einen linearen oder verzweigten organischen Rest mit 1 bis 36 Kohlenstoffatomen steht, der gegebenenfalls Ether-, Ester- und/oder Carbonatgruppen enthalten kann, und
n für 0 oder 1 steht.

3. Verfahren gemäß Anspruch 2, **dadurch gekennzeichnet, dass** als Komponente B) hydroxyfunktionelle cyclische Carbonate der allgemeine Formel (I) oder deren Gemische eingesetzt werden, wobei
R für Wasserstoff oder einen gesättigten linearen aliphatischen Rest mit 1 oder 2 Kohlenstoffatomen steht,
X für einen linearen oder verzweigten organischen Rest mit 1 bis 18 Kohlenstoffatomen steht, der gegebenenfalls Ether-, Ester- und/oder Carbonatgruppen enthalten kann, und
n für 0 oder 1 steht.

4. Verfahren gemäß Anspruch 3, **dadurch gekennzeichnet, dass** als Komponente B) hydroxyfunktionelle cyclische Carbonate der allgemeine Formel (I) oder deren Gemische eingesetzt werden, wobei
R für Wasserstoff oder einen gesättigten linearen aliphatischen Rest mit 1 oder 2 Kohlenstoffatomen steht,
X für eine Methylengruppe (-CH₂-) und
n für 0 oder 1 steht.

5. Verfahren gemäß einem der Ansprüche 1 bis 4, **dadurch gekennzeichnet, dass** als Komponente A) monomere Diisocyanate der allgemeinen Formel (II) oder deren Gemische eingesetzt werden,
**OCN-Y-NCO** (II)
wobei Y für einen linearen oder verzweigten, aliphatischen oder cycloaliphatischen Rest mit 4 bis 18 Kohlenstoffatomen oder einen gegebenenfalls substituierten aromatischen oder araliphatischen Rest mit 6 bis 18 Kohlenstoffatomen steht.

6. Verfahren gemäß Anspruch 5, **dadurch gekennzeichnet, dass** als Komponente A) monomere Diisocyanate der allgemeinen Formel (II) oder deren Gemische eingesetzt werden, wobei Y für einen linearen oder verzweigten, aliphatischen oder cycloaliphatischen Rest mit 6 bis 13 Kohlenstoffatomen steht.

7. Verfahren gemäß Anspruch 6, **dadurch gekennzeichnet, dass** als Komponente A) 1,6-Diisocyanatohexan, 1-Isocyanato-3,3,5-trimethyl-5-isocyanatomethylcyclohexan, 2,4'- und/oder 4,4'-Diisocyanatodicyclohexylmethan oder deren Gemische eingesetzt werden.

8. Verfahren gemäß einem der Ansprüche 1 bis 7, **dadurch gekennzeichnet, dass** die Komponente A) mit Komponente B) in einem Äquivalentverhältnis von Isocyanatgruppen zu Hydroxylgruppen von mindestens 10 : 1, bevorzugt 12 : 1 umgesetzt wird.

9. Verfahren gemäß einem der Ansprüche 1 bis 8, **dadurch gekennzeichnet, dass** die Komponente A) mit Komponente B) in einem Äquivalentverhältnis von Isocyanatgruppen zu Hydroxylgruppen von höchstens 40 : 1, bevorzugt von höchstens 30 : 1 umgesetzt wird.

10. Verfahren gemäß einem der Ansprüche 1 bis 9, **dadurch gekennzeichnet, dass** nach der Umsetzung der Komponenten A) mit B) ein nicht umgesetzter Überschusses an monomeren Diisocyanaten A) bis auf einen Restgehalt von weniger als 1 Gew.-%, bezogen auf die Gesamtmasse des Reaktionsproduktes, vom Reaktionsprodukt abgetrennt wird.

11. Isocyanatgruppen und cyclische Carbonatstukturen enthaltende Verbindungen erhältlich nach einem Verfahren gemäß einem der Ansprüche 1 bis 10.

12. Zusammensetzung enthaltend
a) Isocyanatgruppen und cyclische Carbonatstrukturen enthaltende Verbindungen der allgemeinen Formel (III), 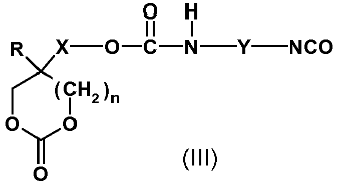
b) Isocyanatgruppen und cyclische Carbonatstukturen enthaltende Verbindungen der allgemeinen Formel (IV) 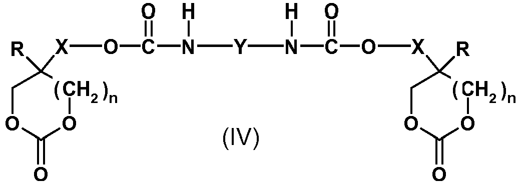 und
c) monomere Diisocyanate mit aliphatisch, cycloaliphatisch, araliphatisch und/oder aromatisch gebundenen Isocyanatgruppen,
wobei in den Formeln (III) und (IV)
R für Wasserstoff oder einen gesättigten oder ungesättigten, linearen oder verzweigten, aliphatischen Rest mit 1 bis 7 Kohlenstoffatomen steht,
X für einen linearen oder verzweigten organischen Rest mit 1 bis 36 Kohlenstoffatomen steht, der gegebenenfalls Ether-, Ester- und/oder Carbonatgruppen enthalten kann,
n für 0 oder 1 steht und
Y für einen linearen oder verzweigten, aliphatischen oder cycloaliphatischen Rest mit 4 bis 18 Kohlenstoffatomen oder einen gegebenenfalls substituierten aromatischen oder araliphatischen Rest mit 6 bis 18 Kohlenstoffatomen steht,
**dadurch gekennzeichnet, dass** jeweils bezogen auf die Gesamtmenge der Komponenten a) und b) die Komponente a) einen Anteil von ≥ 88 Gew.-%, bevorzugt ≥ 90 Gew.-% und die Komponente b) einen Anteil von ≤ 12 Gew.-%, bevorzugt ≤ 10 Gew.-% ausmacht und die Komponente c) zu ≤ 1 Gew.-% in der Gesamtzusammensetzung enthalten ist.

13. Zusammensetzung gemäß Anspruch 12, **dadurch gekennzeichnet, dass** die Komponente b) einen Anteil von 0,5 bis 12 Gew.-%, bevorzugt 0,5 bis 10 Gew.-% bezogen auf die Gesamtmenge der Komponenten a) und b) ausmacht.

14. Verwendung der Isocyanatgruppen und cyclische Carbonatstrukturen enthaltenden Verbindungen gemäß Anspruch 11 oder der Zusammensetzung gemäß einem der Ansprüche 12 oder 13 als Ausgangskomponente bei der Herstellung von cyclische Carbonatstrukturen enthaltenden Polyurethanen oder als Ausgangskomponente bei der Herstellung von vernetzbaren Bindemitteln oder als Ausgangskomponente bei der Herstellung von vernetzbaren Lack-, Dichtstoff- oder Klebstoffrohstoffen.

15. Cyclische Carbonatstrukturen enthaltende Polyurethane, hergestellt unter Verwendung der Isocyanatgruppen und cyclische Carbonatstrukturen enthaltenden Verbindungen gemäß Anspruch 11 oder Zusammensetzungen gemäß einem der Ansprüche 12 oder 13.

## Claims

1. Process for preparing compounds containing isocyanate groups and cyclic carbonate structures, by reacting at least
A) a monomeric diisocyanate having aliphatically, cycloaliphatically, araliphatically and/or aromatically bonded isocyanate groups
with
B) a hydroxy-functional cyclic carbonate,
**characterized in that** component A) is reacted with component B) in a ratio of equivalents of isocyanate groups to hydroxyl groups of at least 8:1.

2. Process according to Claim 1, **characterized in that** hydroxy-functional cyclic carbonates of the general formula (I) or mixtures thereof are used as component B) 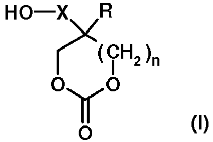 where
R is hydrogen or a saturated or unsaturated, linear or branched, aliphatic radical having 1 to 7 carbon atoms,
X is a linear or branched organic radical which has 1 to 36 carbon atoms and which may optionally contain ether, ester and/or carbonate groups, and
n is 0 or 1.

3. Process according to Claim 2, **characterized in that** hydroxy-functional cyclic carbonates of the general formula (I) or mixtures thereof are used as component B), where
R is hydrogen or a saturated linear aliphatic radical having 1 or 2 carbon atoms,
X is a linear or branched organic radical which has 1 to 18 carbon atoms and which may optionally contain ether, ester and/or carbonate groups, and
n is 0 or 1.

4. Process according to Claim 3, **characterized in that** hydroxy-functional cyclic carbonates of the general formula (I) or mixtures thereof are used as component B), where
R is hydrogen or a saturated linear aliphatic radical having 1 or 2 carbon atoms,
X is a methylene group (-CH₂-) and
n is 0 or 1.

5. Process according to any of Claims 1 to 4, **characterized in that** monomeric diisocyanates of the general formula (II) or mixtures thereof are used as component A)
**OCN-Y-NCO** (II)
where Y is a linear or branched, aliphatic or cycloaliphatic radical having 4 to 18 carbon atoms or an optionally substituted aromatic or araliphatic radical having 6 to 18 carbon atoms.

6. Process according to Claim 5, **characterized in that** monomeric diisocyanates of the general formula (II) or mixtures thereof are used as component A), where Y is a linear or branched, aliphatic or cycloaliphatic radical having 6 to 13 carbon atoms.

7. Process according to Claim 6, **characterized in that** 1,6-diisocyanatohexane, 1-isocyanato-3,3,5-trimethyl-5-isocyanatomethylcyclohexane, 2,4'- and/or 4,4'-diisocyanatodicyclohexylmethane or mixtures thereof are used as component A).

8. Process according to any of Claims 1 to 7, **characterized in that** component A) is reacted with component B) in a ratio of equivalents of isocyanate groups to hydroxyl groups of at least 10:1, preferably 12:1.

9. Process according to any of Claims 1 to 8, **characterized in that** component A) is reacted with component B) in a ratio of equivalents of isocyanate groups to hydroxyl groups of at most 40:1, preferably of at most 30:1.

10. Process according to any of Claims 1 to 9, **characterized in that**, after the reaction of components A) with B), any unconverted excess of monomeric diisocyanates A) is removed from the reaction product down to a residual content of less than 1% by weight, based on the total mass of the reaction product.

11. Compounds containing isocyanate groups and cyclic carbonate structures, obtainable by a process according to any of Claims 1 to 10.

12. Composition comprising
a) compounds of the general formula (III) containing isocyanate groups and cyclic carbonate structures, 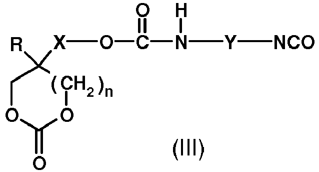
b) compounds of the general formula (IV) containing isocyanate groups and cyclic carbonate structures, 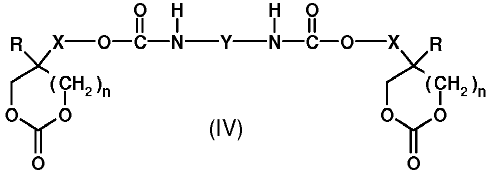 and
c) monomeric diisocyanates having aliphatically, cycloaliphatically, araliphatically and/or aromatically bonded isocyanate groups,
where, in the formulae (III) and (IV),
R is hydrogen or a saturated or unsaturated, linear or branched, aliphatic radical having 1 to 7 carbon atoms,
X is a linear or branched organic radical which has 1 to 36 carbon atoms and which may optionally contain ether, ester and/or carbonate groups,
n is 0 or 1 and
Y is a linear or branched, aliphatic or cycloaliphatic radical having 4 to 18 carbon atoms or an optionally substituted aromatic or araliphatic radical having 6 to 18 carbon atoms,
**characterized in that**, based in each case on the total amount of components a) and b), component a) makes up a proportion of ≥ 88% by weight, preferably ≥ 90% by weight, and component b) a proportion of ≤ 12% by weight, preferably ≤ 10% by weight, and component c) is present to an extent of ≤ 1 % by weight, in the overall composition.

13. Composition according to Claim 12, **characterized in that** component b) makes up a proportion of 0.5% to 12% by weight, preferably 0.5% to 10% by weight, based on the total amount of components a) and b).

14. Use of the compounds containing isocyanate groups and cyclic carbonate structures according to Claim 11 or of the composition according to either of Claims 12 and 13 as a starting component in the production of polyurethanes containing cyclic carbonate structures or as a starting component in the production of crosslinkable binders or as a starting component in the production of crosslinkable raw materials for varnishes, sealants or adhesives.

15. Polyurethanes containing cyclic carbonate structures, prepared using the compounds containing isocyanate groups and cyclic carbonate structures according to Claim 11 or compositions according to either of Claims 12 and 13.

## Revendications

1. Procédé pour la préparation de composés contenant des groupes isocyanate et des structures cycliques de type carbonate, par la transformation d'au moins
A) un diisocyanate monomérique présentant des groupes isocyanates liés de manière aliphatique, cycloaliphatique, araliphatique et/ou aromatique
avec
B) un carbonate cyclique à fonctionnalité hydroxyle,
**caractérisé en ce que** le composant A) mis à réagir avec le composant B) en un rapport d'équivalent de groupes isocyanate à groupes hydroxyle d'au moins 8:1.

2. Procédé selon la revendication 1, **caractérisé en ce que** des carbonates cycliques à fonctionnalité hydroxyle de la formule générale (I) ou des mélanges de ceux-ci sont utilisés en tant que composant B), 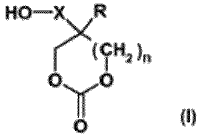
R représentant hydrogène ou un radical aliphatique linéaire ou ramifié, saturé ou insaturé comportant 1 à 7 atome(s) de carbone,
X représentant un radical organique linéaire ou ramifié comportant 1 à 36 atome(s) de carbone, qui peut éventuellement contenir des groupes éther, ester et/ou carbonate et
n représentant 0 ou 1.

3. Procédé selon la revendication 2, **caractérisé en ce que** des carbonates cycliques à fonctionnalité hydroxyle de la formule générale (I) ou des mélanges de ceux-ci sont utilisés en tant que composant B),
R représentant hydrogène ou un radical aliphatique linéaire saturé comportant 1 ou 2 atome(s) de carbone,
X représentant un radical organique linéaire ou ramifié comportant 1 à 18 atome(s) de carbone, qui peut éventuellement contenir des groupes éther, ester et/ou carbonate,
et
n représentant 0 ou 1.

4. Procédé selon la revendication 3, **caractérisé en ce que** des carbonates cycliques à fonctionnalité hydroxyle de la formule générale (I) ou des mélanges de ceux-ci sont utilisés en tant que composant B),
R représentant hydrogène ou un radical aliphatique linéaire saturé comportant 1 ou 2 atome(s) de carbone,
X représentant un groupe méthylène (-CH₂-) et
n représentant 0 ou 1.

5. Procédé selon l'une quelconque des revendications 1 à 4, **caractérisé en ce que** des diisocyanates monomériques de la formule générale (II) ou des mélanges de ceux-ci sont utilisés en tant que composant A),
**OCN-Y-NCO** (II)
Y représentant un radical aliphatique ou cycloaliphatique, linéaire ou ramifié comportant 4 à 18 atomes de carbone ou un radical aromatique ou araliphatique éventuellement substitué comportant 6 à 18 atomes de carbone.

6. Procédé selon la revendication 5, **caractérisé en ce que** des diisocyanates monomériques de la formule générale (II) ou des mélanges de ceux-ci sont utilisés en tant que composant A), Y représentant un radical aliphatique ou cycloaliphatique, linéaire ou ramifié comportant 6 à 13 atomes de carbone.

7. Procédé selon la revendication 6, **caractérisé en ce que** le 1,6-diisocyanatohexane, le 1-isocyanato-3,3,5-triméthyl-5-isocyanatométhylcyclohexane, le 2,4'-diisocyanatodicyclohexylméthane et/ou le 4,4'-diisocyanatodicyclohexylméthane ou des mélanges de ceux-ci sont utilisés en tant que composant A).

8. Procédé selon l'une quelconque des revendications 1 à 7, **caractérisé en ce que** le composant A) mis à réagir avec le composant B) en un rapport d'équivalent de groupes isocyanate à groupes hydroxyle d'au moins 10:1, préférablement de 12:1.

9. Procédé selon l'une quelconque des revendications 1 à 8, **caractérisé en ce que** le composant A) mis à réagir avec le composant B) en un rapport d'équivalent de groupes isocyanate à groupes hydroxyle d'au plus 40:1, préférablement d'au plus 30:1.

10. Procédé selon l'une quelconque des revendications 1 à 9, **caractérisé en ce qu'**après la transformation du composant A) avec B), un excès de diisocyanate monomérique A) qui n'a pas réagi est séparé du produit de réaction jusqu'à une teneur résiduelle inférieure à 1 % en poids, par rapport à la masse totale du produit de réaction.

11. Composés contenant des groupes isocyanate et des structures cycliques de type carbonate pouvant être obtenus par un procédé selon l'une quelconque des revendications 1 à 10.

12. Composition contenant
a) des composés contenant des groupes isocyanate et des structures cycliques de type carbonate de la formule générale (III), 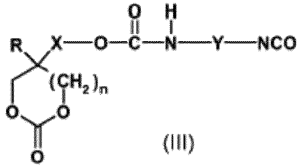
b) des composés contenant des groupes isocyanate et des structures cycliques de type carbonate de la formule générale (IV), 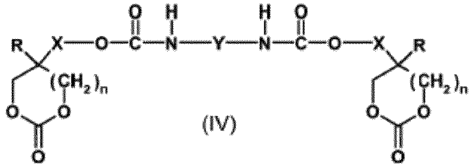 et
c) des diisocyanates monomériques présentant des groupes isocyanates liés de manière aliphatique, cycloaliphatique, araliphatique et/ou aromatique,
et dans les formules (III) et (IV)
R représentant hydrogène ou un radical aliphatique linéaire ou ramifié, saturé ou insaturé comportant 1 à 7 atome(s) de carbone,
X représentant un radical organique linéaire ou ramifié comportant 1 à 36 atome(s) de carbone, qui peut éventuellement contenir des groupes éther, ester et/ou carbonate,
n représentant 0 ou 1 et
Y représentant un radical aliphatique ou cycloaliphatique, linéaire ou ramifié comportant 4 à 18 atomes de carbone ou un radical aromatique ou araliphatique éventuellement substitué comportant 6 à 18 atomes de carbone,
**caractérisée en ce que** le composant a) représente une part ≥ 88% en poids, préférablement ≥ 90% en poids et le composant b) représente une part ≤ 12% en poids, préférablement ≤ 10% en poids, par rapport à la quantité totale du composant a) et du composant b) et le composant c) est contenu dans la composition totale à raison de ≤ 1 % en poids.

13. Composition selon la revendication 12, **caractérisée en ce que** le composant b) représente une part de 0,5 à 12% en poids, préférablement de 0,5 à 10% en poids par rapport à la quantité totale des composants a) et b).

14. Utilisation des composés contenant des groupes isocyanate et des structures cycliques de type carbonate selon la revendication 11 ou de la composition selon l'une quelconque des revendications 12 ou 13 en tant que composant de départ lors de la préparation de polyuréthanes contenant des structures cycliques de type carbonate ou en tant que composant de départ lors de la préparation de liants réticulables ou en tant que composant de départ lors de la préparation de matières premières réticulables de laques, de matières premières réticulables de matériaux d'étanchéité ou de matières premières réticulables d'adhésifs.

15. Polyuréthanes contenant des structures cycliques de type carbonate, préparés en utilisant les composés contenant des groupes isocyanate et des structures cycliques de type carbonate selon la revendication 11 ou des compositions selon l'une quelconque des revendications 12 ou 13.
